# NEUE EUROPÄISCHE PATENTSCHRIFT

(11) **EP 3 042 645 B2**
(45) Veröffentlichungstag und Bekanntmachung des Hinweises auf die Entscheidung über den Einspruch: **12.03.2025**
(45) Hinweis auf die Patenterteilung: 27.04.2022
(21) Anmeldenummer: 15150607.8
(22) Anmeldetag: 09.01.2015
(51) Int. Cl.: A61K 6/61, A61K 6/62, A61K 6/887

(54) **Dentalkomposite mit verbesserter Lagerstabilität**
Dental composites with improved storage stability
Composite dentaire avec stabilité au stockage améliorée

(43) Veröffentlichungstag der Anmeldung: 13.07.2016
(73) Patentinhaber: Ivoclar Vivadent AG, 9494 Schaan (LI)
(72) Erfinder: Moszner, Norbert, 9495 Triesen (LI); Fischer, Urs-Karl, 9320 Arbon (CH); Schubert, Ulrich, 07743 Jena (DE); Hager, Martin D., 07774 Dornburg-Camburg (DE)
(74) Vertreter: Uexküll & Stolberg

(56) Entgegenhaltungen:
- EP-B1- 0 290 133
- EP-B1- 1 819 313
- WO-A1-01/44338
- WO-A2-2006/069394
- JP-A- 2013 095 690
- US-A- 4 889 792
- US-A1- 2005 009 946
- US-B1- 6 444 725
- US-B2- 8 367 051
- DATABASE WPI Week 201479, Derwent World Patents Index; AN 2014-U44635, XP002741394
- DATABASE WPI Week 201338, Derwent World Patents Index; AN 2013-H51064, XP002741395
- K. MATYJASZEWSKI ET AL: "Diminishing catalyst concentration in atom transfer radical polymerization with reducing agents", PROCEEDINGS OF THE NATIONAL ACADEMY OF SCIENCES, vol. 103, no. 42, 17 October 2006 (2006-10-17), pages 15309 - 15314, XP055176931, ISSN: 0027-8424, DOI: 10.1073/pnas.0602675103
- FORBES DIANE C., PEPPAS NICHOLAS A.: "Polymeric Nanocarriers for siRNA Delivery to Murine Macrophages : Polymeric Nanocarriers for siRNA Delivery", MACROMOLECULAR BIOSCIENCE, WILEY-VCH VERLAG GMBH, DE, vol. 14, no. 8, 1 August 2014 (2014-08-01), DE , pages 1096 - 1105, ISSN: 1616-5187, DOI: 10.1002/mabi.201400027
- ANONYMOUS: "Clinpro Sealant Technical Product Profile", 3M ESPE, 1 January 2001 (2001-01-01), pages 1 - 20
- LI WENWEN ET AL: "Atom Transfer Radical Copolymerization of Monomer and Cross-Linker under Highly Dilute Conditions", MACROMOLECULES, AMERICAN CHEMICAL SOCIETY, US, vol. 44, no. 9, 10 May 2011 (2011-05-10), US , pages 3270 - 3275, ISSN: 0024-9297, DOI: 10.1021/ma200192e
- VAN CAMP WIM ET AL: "Effect of crosslinker multiplicity on the gel point in ATRP", JOURNAL OF POLYMER SCIENCE PART A: POLYMER CHEMISTRY, JOHN WILEY & SONS, INC., US, vol. 48, no. 9, 1 May 2010 (2010-05-01), US , pages 2016 - 2023, ISSN: 0887-624X, DOI: 10.1002/pola.23970

## Beschreibung

Die vorliegende Erfindung betrifft redox-chemisch oder dualhärtende Komposite zur Herstellung von Dentalwerkstoffen wie dentalen Zementen zur Befestigung von Kronen, Brücken, Inlays oder Onlays, und Füllungsmaterialien.

Dentale Komposite, die z.B. als Kompositzement oder als direktes Füllungsmaterial, Inlay, Onlay, Krone oder Verblendwerkstoff eingesetzt werden, enthalten eine polymerisierbare organische Matrix und einen oder mehreren Füllstoffe, die meist mit einem polymerisationsfähigen Haftvermittler oberflächenmodifiziert sind. Je nach Art der Füllstoffe, der Monomermatrix und der gewünschten Anwendung kann der Füllungsgrad zwischen ca. 50-90 Gew.-% variieren, wobei Zemente im Vergleich zu Füllungskompositen einen geringeren Füllungsgrad aufweisen.

Die polymerisierbare organische Matrix enthält in der Regel eine Mischung von Monomeren, Initiatorkomponenten, Stabilisatoren und Pigmenten. Als Monomere werden meistens Mischungen von Dimethacrylaten eingesetzt. Beispiele hierfür sind die hochviskosen Dimethacrylate 2,2-Bis[4-(2-hydroxy-3-methacryl-oyloxypropyl)phenyl]propan (Bis-GMA) und 1,6-Bis-[2-methacryl-oyloxyethoxycarbonylamino]-2,4,4-trimethylhexan (UDMA) sowie die als Verdünnermonomere genutzten niedrigerviskosen Dimethacrylate, wie z.B. Bismethacryloyloxymethyltricyclo-[5.2.1.]decan (TCDMA), Decandiol-1,10-dimethacrylate (D₃MA) und Triethylenglycoldimethacrylat (TEGDMA).

Bei der Aushärtung von dentalen Kompositen auf Dimethacrylatbasis kommen unterschiedliche radikalbildende Initiatoren zum Einsatz. Lichthärtende Komposite enthalten häufig Campherchinon (CQ) als Photoinitiator, der Licht im blauen Bereich des sichtbaren Spektrums (400-500 nm) absorbiert. Zur Beschleunigung des lichtinduzierten Zerfalls des CQ werden tertiäre Amine wie z.B. EMBO (4-Dimethylaminobenzoesäureethylester) als Coinitiatoren eingesetzt, aus denen sich polymerisationsauslösenden Aminoalkylradikale bilden.

Neben Photoinitiatoren sind redox-chemisch härtende Initiatorsysteme gebräuchlich, die aus einem Oxidationsmittel, meist einem Peroxid wie Dibenzoylperoxid (DBPO), und einem Reduktionsmittel bestehen. Als Reduktionsmittel haben sich tertiäre aromatische Amine wie N,N-Diethanol-p-toluidin oder N,N-Diethyl-3,5-di-tert.-butylanilin bewährt. Diese Reduktionsmittel beschleunigen die radikalbildende Zersetzung des DBPO, so dass eine Polymerisationsauslösung bei Raumtemperatur möglich ist. Bei Kompositen, die acide Monomere enthalten, sind aminfreie Redoxsysteme bevorzugt, weil Säuren zur Neutralisation der basischen Aminbeschleuniger führen. Aminfreie Reduktionsmittel, die sich zur Kombination mit Peroxiden und Hydroperoxiden eignen, sind z.B. Ascorbinsäure, p-Toluolsulfinsäure und Trimethylbarbitursäure.

Redoxsysteme werden immer dann eingesetzt, wenn, wie z.B. bei Befestigungskompositen, mangels ausreichender Durchstrahlung die Lichtinitiierung nicht angewendet werden kann. Im Vergleich zur Lichthärtung erfolgt die Aushärtung deutlich langsamer. Befestigungskomposite enthalten oft auch sogenannte dualhärtende Initiatorsysteme. In der Regel handelt es sich dabei um eine Kombination aus einem Photoinitiator und einem Redoxinitiatorsystem.

Peroxide haben eine stark begrenzte thermische Stabilität und können in Anwesenheit von Verunreinigungen spontan explodieren. Um sie dennoch praktisch nutzen zu können, setzt man zur Stabilisierung sogenannte Phlegmatisierungsmittel wie z.B. Phthalate zu, die aber zu toxikologischen Problemen führen können. Peroxid-Redoxinitiatorsysteme und insbesondere die in Dentalwerkstoffen häufig verwendeten DBPO-Redoxsysteme haben den weiteren Nachteil, dass sie nur eine begrenzte Lagerstabilität aufweisen und deshalb gekühlt gelagert und transportiert werden müssen.

Die sogenannte Atom Transfer Radical Polymerization (ATRP) kommt ohne Peroxide aus. Hier wird eine Kombination von Übergangsmetallkomplexen, vor allem Kupfer-, Ruthenium- oder Eisenkomplexen, mit Organohalogenverbindungen zur Initiierung der radikalischen Polymerisation eingesetzt (N. Ayres, Polym. Rev. 51 (2011) 138-162; T Ando, M. Kamigaito, M. Sawamoto, Macromolecules, 2000, 33, 5825-5829). Ein Nachteil der ATRP ist, dass relativ große Metallmengen notwendig sind und dass die Metallkatalysatoren leicht oxidiert werden. Die erforderliche Übergangsmetallkonzentration kann durch Zugabe von Reduktionsmitteln, wie z.B. Zinn(II)-2-ethylhexanoat oder Glucose, verringert werden (W. Jakubowski, K. Matyjazewski, Angew. Chem. 118 (2006) 45). Bei Zugabe bestimmter Photoinitiatoren wie Bis-(2,4,6-trimethylbenzoxyl)phenylphosphinoxid oder bestimmter Farbstoffe wie Eosin Y und Erythrosin B kann die ATRP auch direkt oder indirekt durch Licht induziert werden (M. A. Tasdelen, M. Ciftci, Y. Yagci, Macromol. Chem. Phys. 213 (2012) 1391-1396).

Die ATRP ist ein Multikomponentensystem, das neben dem Monomer, einen Initiator mit einer übertragbaren Halogengruppe und einen Katalysator enthält, der sich aus einem Übergangsmetall und geeigneten Liganden zusammensetzt. Das primäre Ziel der ATRP ist es, eine kontrollierte radikalische Polymerisation zu erreichen, d.h. eine Kontrolle der Molmasse und die Erzielung einer engen Molmasseverteilung. Da bei der vernetzenden Polymerisation eine Kontrolle der Molmasse nicht möglich ist, weil diese durch die Vernetzung schnell auf unendliche Werte ansteigt, wurden bisher praktisch ausschließlich nicht vernetzende Monomere, d.h. Monomere mit nur einer radikalisch polymerisierbaren Gruppe untersucht. Dentalwerkstoffe, die durch ATRP gehärtet werden können, sind nicht bekannt.

Der Erfindung liegt die Aufgabe zugrunde, Dentalwerkstoffe und insbesondere dentale Komposite bereitzustellen, die sich im Vergleich zu bekannten Materialien auf der Basis von Dimethacrylaten durch eine verbesserte Lagerstabilität bei Raumtemperatur auszeichnen, die keine Phlegmatisierungsmittel enthalten und die redox-chemisch schnell aushärten.

Die Aufgabe wird erfindungsgemäß durch Dentalwerkstoffe gelöst, die
(a) 5 bis 80 Gew.-% mindestens eines di- oder polyfunktionellen radikalisch polymerisierbaren (Meth)acrylatmonomers,
(b) 0,1 bis 6,0 Gew.-% mindestens einer organischen Halogenverbindung,
(c) 0,0001 bis 1.0 Gew.-% mindestens einer Übergangsmetallverbindung,
(d) 0,01 bis 5,0 Gew.-% mindestens eines Reduktionsmittels und
(e) 10 bis 85 Gew.-% mindestens eines organischen und/oder anorganischen Füllstoffs enthalten. Die Werkstoffe enthalten keine Peroxide.
Wenn der Bestandteil (c) ein nichtkomplexes Übergangsmetallsalz ist, enthalten die Dentalwerkstoffe zusätzlich 0,0001 bis 1,0 Gew.-% mindestens einer komplexierenden organischen Verbindung.

Die erfindungsgemäßen Dentalwerkstoffe enthalten als Komponente (a) mindestens ein radikalisch polymerisierbares Monomer, vorzugsweise eine Mischung radikalisch polymerisierbarer Monomere, d.h. ein oder mehrere (Meth)acrylate, besonders bevorzugt eine Mischung aus mono- und polyfunktionellen Methacrylaten, ganz besonders bevorzugt aus mono- und difunktionellen Methacrylaten. Dabei sind solche Dentalwerkstoffe bevorzugt, die als radikalisch polymerisierbare Monomere ausschließlich mono- und polyfunktionelle Methacrylate enthalten. Unter monofunktionellen Monomeren werden Verbindungen mit einer, unter polyfunktionellen Monomeren Verbindungen mit zwei oder mehr, vorzugsweise 2 bis 4 radikalisch polymerisierbaren Gruppen verstanden.

Bevorzugte mono- oder polyfunktionelle Methacrylate sind Methyl-, Ethyl-, 2-Hydroxyethyl-, Butyl-, Benzyl-, Tetrahydrofurfuryl- oder Isobornyl(meth)acrylat, p-Cumyl-phenoxyethylenglycolmethacrylat (CMP-1E), Bisphenol-A-dimethacrylat, Bis-GMA (ein Additionsprodukt aus Methacrylsäure und Bisphenol-A-diglycidylether), ethoxy- oder propoxyliertes Bisphenol-A-Di-methacrylat, wie z.B. das Bisphenol-A-Dimethacrylat SR-348c mit 3 Ethoxygruppen oder 2,2-Bis[4-(2-methacryloxypropoxy)phe-nyl]propan, UDMA (ein Additionsprodukt aus 2-Hydroxyethyl-methacrylat (HEMA) und 2,2,4-Trimethylhexamethylendiiso-cyanat), TMX-UDMA (ein Additionsprodukt aus einer Mischung von HEMA und Hydroxypropylmethacrylat (HPMA) mit α,α,α',α'-Tetra-methyl-m-xylylendiisocyanat (TMXDI), Di-, Tri- oder Tetra-ethylenglycoldimethacrylat, Trimethylolpropantrimethacrylat, Pentaerythrittetramethacrylat, sowie Glycerindi- und -trimeth-acrylat, 1,4-Butandioldimethacrylat, 1,10-Decandioldimeth-acrylat (D₃MA) oder 1,12-Dodecandioldimethacrylat. Besonders geeignet sind Mischungen aus CMP-1E, UDMA, und TMX-UDMA sowie Glycerindi- bzw. Glycerintrimethacrylat und/oder D₃-MA.

Erfindungsgemäß wird vorzugsweise eine Monomermischung verwendet, die mindestens ein schwerflüchtiges Monomethacrylat, mindestens ein hochviskoses poly-, vorzugsweise difunktionelles Methacrylat und mindestens ein niedrigviskoses poly-, vorzugsweise difunktionelles Methacrylat enthält.

Erfindungsgemäß werden unter schwerflüchtigen Monomeren Verbindungen mit einem Siedepunkt > 150 °C bei Normaldruck verstanden. Der Siedepunkt kann z.B. mit einer Destillationsapparatur bestimmt werden. Unter hochviskosen Monomeren werden Stoffe mit einer Viskosität ≥ 5 Pa·s, vorzugsweise 5 bis 10.000 Pa·s und besonders bevorzugt 5 bis 2.000 Pa·s, und unter niedrigviskosen Monomeren Stoffe mit einer Viskosität ≤ 300 mPa·s, vorzugsweise 1 bis 300 mPa·s und besonders bevorzugt 30 bis 300 mPa·s verstanden, wobei die Viskosität mit einem Kapillar- (niedrigviskos) oder Rotationsviskosimeter (hochviskos) bei einer Temperatur von 25°C bestimmt wird.

Besonders bevorzugte hochviskosen Dimethacrylate sind TMX-UDMA (ein Additionsprodukt von HEMA und Hydroxypropylmethacrylat (HPMA) mit α,α,α',α'-Tetramethyl-m-xylylendiisocyanat (TMXDI)) und 1,6-Bis-[2-methacryloyloxyethoxycarbonylamino]-2,4,4-tri-methylhexan (UDMA). Bevorzugte niedrigviskose Dimethacrylate, die als Verdünnermonomere genutzten werden, sind Bismethacryl-oyloxymethyltricyclo[5.2.1.]decan (TCDMA), Glycerindimethacrylat (GDMA) und insbesondere Decandiol-1,10-dimethacrylat (D₃MA). Ein besonders bevorzugtes schwerflüchtiges Monomethacrylat ist p-Cumyl-phenoxyethylenglycolmethacrylat (CMP-1E).

Die organische Halogenverbindung (b) fungiert als Initiator für die Atom Transfer Radical Polymerization (ATRP). Sie verfügt über mindestens ein übertragbares Halogenatom und bildet bei der Reaktion mit dem Übergangsmetallkomplex (c) durch die Übertragung des Halogenatoms auf den Metallkomplex Radikale. Der Übergangsmetallkomplex (c) wirkt als Katalysator.

Bevorzugte organische Halogenverbindungen (b) sind:
Halogenierte Alkylaromaten, wie z.B. Benzylhalogenide, insbesondere Benzylchlorid oder -bromid, 1-Phenylethylchlorid, Benzotrichlorid und Benzhydrylchlorid oder -bromid.

α-Halogencarbonsäureester, insbesondere C₁-C₆-Alkylester einer α-Halogen-C₁-C₆-Carbonsäure, wie α-Chlorpropionsäure, α-Brom-propionsäure, α-Chlor-iso-butansäure, α-Brom-iso-butansäure, α-Bromphenylessigsäure. Besonders bevorzugt sind Ester der α-Brom-iso-butansäure wie α-Brom-isobutansäuremethyl- und -ethylester, α-Brom-iso-butyrylbromid, 2-(2-Brom-isobutyryl-oxy)ethylmethacrylate, tert-Butyl-α-brom-iso-butyrat oder 3-Butynyl-2-brom-iso-butyrat, Dipentaerythritolhexakis(2-brom-iso-butyrat) oder 1,1,1-Tris(2-brom-iso-butyryloxymethyl)ethan oder Ethyl α-bromophenylacetat.

α-Halogenketone, wie z.B. 1,1,1-Trichlorpropan-2-on oder Dichlormethylphenylketon.

α-Halogennitrile, wie z.B. 2-Brompropionitril oder Trichloracetonitril.

Sulfonylhalogenide, Methyl-, Trichlormethyl-, p-Toluol- oder 4-Methoxyphenylsulfonylchlorid.

Halogenverbindungen, die als übertragbare Halogenatome Chlor oder Brom enthalten, sind bevorzugt.

Als Übergangsmetallverbindung (c) sind Übergangsmetallkomplexverbindungen bevorzugt, insbesondere Übergangsmetallkomplexverbindungen der Metalle Kupfer, Eisen, Ruthenium, Nickel und Palladium. Anstelle der Übergangsmetallkomplexe können nichtkomplexe Salze der Übergangsmetalle in Kombination mit komplexbildenden organischen Verbindungen zur Herstellung der Dentalwerkstoffe eingesetzt werden. Die organischen Liganden bilden beim Mischen mit den Übergangsmetallsalzen die katalytisch wirksamen Komplexe. Die Verwendung solcher Kombinationen von Übergangsmetallsalzen und organischen Liganden ist bevorzugt.

Als komplexierende organische Verbindungen sind P- und insbesondere N-haltigen Liganden bevorzugt. Bevorzugte Beispiele hierfür sind Phosphine, vorzugsweise Triphenylphosphin; Ethylendiaminotetraessigsäure (EDTA); Phenanthroline, vorzugsweise 1,10-Phenanthrolin (Phen); Bipyridine, vorzugsweise 2,2'-Bipyridin (Bipy), 4,4'-Dimethyl-2,2'-bipyridin, 6,6'-Dimethyl-2,2'-bipyridin und 4,4'-Di(5-nonyl)-2,2'-bipyridin (dNbpy); Terpyridine, vorzugsweise 2':6',2"-Terpyridin (tpy), 4,4',4"-Tris(5-nonyl)- 2,2':6',2"-terpyridin; Pyridinimine, vorzugweise N-Octyl-2-pyridylmethanimin; alkylierte Imidazole, vorzugsweise 1,3-Diisopropyl-4,5-dimethylimidazol-2-yliden (PriIm); alkylaminosubstituierte Benzole, vorzugsweise o,o' (CH₂NMe₂)₂C₆H₃; aliphatische Amine, vorzugsweise 1,1,4,7,10,10-Hexamethyltriethylentetramin (HMTETA), N,N,N',N",N"-Pentamethyldiethylentriamin (PMDETA), Tris[2-(di-methylamino)ethyl]amin (Me₆TREN), N,N,N',N'-Tetramethylethylen-diamin (TMEDA), 1,4,8,11-Tetraaza-1,4,8,11-tetramethylcyclo-tetradecan (Me4CYCLAM), Diethylentriamin (DETA), Triethylentetramin (TETA) und 1,4,8,11-Tetraazacyclotetradecan (CYCLAM)); pyridinhaltige Liganden, vorzugsweise N,N,N',N'-Tetrakis(2-pyridylmethyl)ethylendiamin (TPEN), N,N-Bis(2-pyridylme-thyl)amin (BPMA) und N,N-Bis(2-pyridylmethyl)octylamin (BPMOA), alkylsubstituierte 2-(1H-1,2,3-Triazol-4-yl)pyridine, 2,6-Bis(1H-1,2,3-triazol-4-yl)-pyridin und alkylsubstituierte 2,6-Bis(1H-1,2,3-triazol-4-yl)pyridine.

Bevorzugte Kupfersalze sind CuCl, CuBr, CuCl₂, CuBr₂, CuI₂, Cu(II)-carboxylate (z.B. der Essigsäure oder 2-Ethylhexansäure). Bevorzugte Kupferkomplexe sind Komplexe mit den Liganden Phenanthrolin (z.B. 1,10-Phenanthrolin (Phen)), Terpyridin, Bipyridin (z.B. 2,2'-Bipyridin (Bipy), 4,4'-Dimethyl-2,2'-bipyridin oder 6,6'-Dimethyl-2,2'-bipyridin), Pyridinimin oder aliphatischen Aminen, wie z.B. 1,1,4,7,10,10-Hexamethyltriethylentetramin (HMTETA), N,N,N',N",N"-Penta-methyldiethylentriamin (PMDETA), Tris[2-(dimethylamino)ethyl]-amin (Me₆TREN).

Bevorzugte Eisensalze sind FeCl₃, FeBr₂ und FeCl₂. Bevorzugte Eisenkomplexe sind Komplexe mit den Liganden Triphenylphosphin, 4,4'-Di(5-nonyl)-2,2'-bipyridin (dNbpy) oder 1,3-Diisopropyl-4,5-dimethylimidazol-2-yliden (PriIm). Ganz besonders bevorzugt sind die Komplexe FeCl₂(PPh₃)₂, FeBr₂-dNbpy, FeCl₂(PriIm)₂ und FeBr₂(PriIm)₂.

Ein bevorzugtes Rutheniumsalz ist RuCl₂, bevorzugte Rutheniumkomplexe sind RuCl₂(PPh₃)₄ und RuH₂(PPh₃)₄.

Bevorzugte Nickelsalze sind NiBr₂ und NiCl₂, bevorzugte Nickelkomplexe sind Ni [o,o' (CH₂NMe₂) ₂C₆H₃] Br und NiBr₂ (PPh₃)₂.

Ein bevorzugtes Palladiumsalz ist Pd(OAc)₂.

In allen Fällen sind solche Komplexe bevorzugt, in denen das jeweilige Übergangsmetall in seiner stabilsten Oxidationsstufe vorliegt. Bevorzugt sind damit Komplexe von Cu²⁺, Fe³⁺, Ni²+, Ru³⁺ und Pd²⁺.

Erfindungsgemäß sind Kupferverbindungen, Kupferkomplexe und insbesondere Mischungen von Kupfersalzen und komplexierenden organischen Liganden besonders bevorzugt.

Bei der Verwendung einer Kombination von nichtkomplexem Übergangsmetallsalz und komplexierender organischer Verbindung werden Übergangsmetallsalz und Ligand vorzugsweise in einem molaren Verhältnis 1:1 bis 1:100, besonders bevorzugt 1:5 bis 1:100 eingesetzt. Die für die Auslösung der Polymerisation erforderliche Komplexe bilden sich beim Mischen von Übergangsmetallsalz und Ligand *in situ.*

Bevorzugte Reduktionsmittel (d) sind Zinnverbindungen, wie Sn(II)-octoat oder Sn(II)-2-ethylhexanoat, reduzierende Zucker, z.B. Glucose oder Fructose, Antioxidantien, wie z.B. Ascorbinsäure (Vitamin C) und deren Derivate (bevorzugte Derivate der Ascorbinsäure sind deren Salze und Ester), β-Karotinoide (vorzugsweise Vitamin A), α-Tocopherol (Vitamin E), phenolische Reduktionsmittel, wie z.B. Butylhydroxytoluol (BHT), Butylhydroxyanisol (BHA), Propyl- oder Octylgallat, Pyrogallol, Sulfite, Bisulfite, Thiosulfate, Hydroxylamin und Hydrazin sowie Derivate des Hydroxylamins und des Hydrazins, d.h. insbesondere N-Substitutionsprodukte des Hydroxylamins und des Hydrazins, oder eine Kombination von verschiedenen Reduktionsmitteln. Besonders bevorzugt sind Sn(II)-Verbindungen und Ascorbinsäure sowie deren Ester und Salze.

Die erfindungsgemäßen Dentalwerkstoffe liegen in Form von Kompositen vor. Unter Kompositen werden Zusammensetzungen verstanden, die neben den oben genannten Bestandteilen mindestens einen organischen oder vorzugsweise anorganischen Füllstoff (Bestandteil e) enthalten.

Besonders geeignet sind Füllstoffe auf der Basis von Oxiden mit einer Partikelgröße von 0,01 bis 15 µm, wie SiO₂, ZrO₂ und TiO₂ bzw. Mischoxiden aus SiO₂, ZrO₂, ZnO und/oder TiO₂; nanopartikuläre Füllstoffe mit einer Partikelgröße von 5 bis 300 nm, vorzugsweise 10 bis 300 nm, wie pyrogene Kieselsäure oder Fällungskieselsäure; sowie Glaspulver mit einer Partikelgröße von 0,01 bis 15 µm, vorzugweise von 0,2 bis 1,5 µm, wie Quarz-, Glaskeramik- oder röntgenopake Glaspulver z.B. von Bariumoder Strontiumaluminiumsilikatgläsern; und röntgenopake Füllstoffe mit einer Partikelgröße von 0,2 bis 5 µm, wie Ytterbiumtrifluorid, Tantal(V)-oxid, Bariumsulfat oder Mischoxide von SiO₂ mit Ytterbium(III)-oxid oder Tantal(V)-oxid. Faserförmige Füllstoffe, Nanofasern oder Whiskers sind nicht ausgeschlossen, partikuläre Füller sind jedoch bevorzugt. Wenn nicht anders angegeben handelt es sich bei allen Partikelgrößen um gewichtsmittlere Partikelgrößen.

Die Füllstoffe werden nach der Partikelgröße auch in Makrofüller und Mikrofüller unterteilt. Unter Makrofüllern werden hier Füllstoffe mit einer Partikelgröße von 0,5 µm bis 15 µm und unter Mikrofüllern Füllstoffe mit einer Partikelgröße von 5 bis 300 nm verstanden. Die erfindungsgemäßen Dentalwerkstoffe enthalten vorzugsweise eine Mischung von Makro- und Mikrofüllstoffen. Bevorzugt sind Makrofüller mit einer mittleren Partikelgröße von 0,5 bis 10 µm. Die Mikrofüller haben vorzugsweise eine mittlere Partikelgröße von 5 bis 100 nm.

Makrofüller werden durch Mahlen beispielsweise von Quarz, röntgenopaken Gläsern, Borosilikaten oder von Keramik gewonnen, sind vorzugsweise rein anorganischer Natur und bestehen meist aus splitterförmigen Teilchen. Als Mikrofüller werden vorzugsweise pyrogenes SiO₂ oder Fällungskieselsäure eingesetzt, oder auch Mischoxide, z.B. SiO₂-ZrO₂, die durch hydrolytische Cokondensation von Metallalkoxiden zugänglich sind.

Außerdem können sogenannte Kompositfüller als Makrofüller verwendet werden. Hierbei handelt es sich um partikuläre, beispielsweise splitterförmige, mikrogefüllte Polymerisate, die z.B. durch Einarbeiten von pyrogenem SiO₂ und/oder röntgenopaken Glasfüllstoffen in eine Harzmatrix, anschließende thermische Polymerisation der Mischung und Mahlen des so erhaltenen Polymerisates zugänglich sind. Kompositfüller werden auch als Isofüller bezeichnet.

Zur Verbesserung des Verbundes zwischen den Füllstoffpartikeln und der vernetzten Polymerisationsmatrix können SiO₂-basierende Füllstoffe mit (Meth)acrylat-funktionalisierten Silanen oberflächenmodifiziert werden. Ein bevorzugtes Beispiel für solche Silane ist 3-(Meth)acryloyloxypropyltrimethoxysilan. Zur Oberflächenmodifizierung von nichtsilikatischen Füllstoffen wie z.B. von ZrO₂ oder TiO₂ können auch funktionalisierte saure Phosphate, wie z.B. 10-(Meth)acryloyloxydecyldihydrogenphos-phat eingesetzt werden.

Der Füllungsgrad richtet sich nach dem gewünschten Anwendungszweck. Füllungskomposite haben vorzugsweise einen Füllstoffgehalt von mindestens 75 Gew.-% und Kompositzemente vorzugsweise von 40-75 Gew.-%, vorzugsweise 50 bis 60 Gew.-%.

Gemäß einer bevorzugten Ausführungsform enthalten die Dentalwerkstoffe als Bestandteil (f) vorzugsweise zusätzlich mindestens einen Photoinitiator für die radikalische Polymerisation. Durch die Zugabe eines Photoinitiators können dualhärtende Werkstoffe erhalten werden, wobei als Photoinitiatoren α-Diketone, wie 9,10-Phenanthrenchinon, 1-Phenyl-propan-1,2-dion, Diacetyl oder 4,4'-Dichlorbenzil, bevorzugt sind. Besonders bevorzugt werden Campherchinon (CQ) und 2,2-Dimethoxy-2-phenyl-acetophenon und ganz besonders bevorzugt α-Diketone jeweils in Kombination mit einem Amin, wie z.B. 4-(Dimethyl-amino)-benzoesäureester (EMBO), N,N-Dimethylaminoethylmeth-acrylat, N,N-Dimethyl-sym.-xylidin oder Triethanolamin, als Reduktionsmittel verwendet. Weiter bevorzugt sind Norrish-Typ-I-Photoinitiatoren, vor allem Acyl- oder Bisacylphosphinoxide, und insbesondere Monoacyltrialkyl- bzw. Diacyldialkylger-manium-Verbindungen, wie z.B. Benzoyltrimethylgermanium, Dibenzoyldiethylgermanium oder Bis(4-methoxybenzoyl)diethyl-germanium (MBDEGe). Mischungen dieser Photoinitiatoren sind ebenfalls geeignet, wie z.B. Bis(4-methoxybenzoyl)-diethylgermanium in Kombination mit CQ/EMBO. Dual härtende Werkstoffe eignen sich besonders als Befestigungszemente.

Außerdem können die erfindungsgemäßen Zusammensetzungen weitere Additive (Bestandteil g) enthalten, vor allem Stabilisatoren, Farbmittel, mikrobiozide Wirkstoffe, fluoridionenabgebende Additive, Treibmittel, optische Aufheller und/oder UV-Absorber. Als Stabilisatoren eignen sich besonders aerobe Stabilisatoren, z.B. Phenole wie BHT oder MEHQ (Hydrochinonmonomethylether), und anaerobe Stabilisatoren, wie Phenothiazin oder N,N'-Diphenyl-1,4-phenylendiamin.

Die erfindungsgemäßen Werkstoffe enthalten keine Peroxide und vorzugsweise auch keine Weichmacher und/oder Phlegmatisierungsmittel, d.h. insbesondere keine Phthalate. Weiterhin ist es bevorzugt, dass die erfindungsgemäßen Werkstoffe keine aciden Monomere enthalten. In allen Ausführungsformen sind die erfindungsgemäßen Werkstoffe vorzugsweise lösungsmittelfrei.

Die erfindungsgemäßen Dentalwerkstoffe enthalten vorzugsweise:
(a) 10 bis 70 Gew.-% und besonders bevorzugt 15 bis 60 Gew.-% polyfunktionelle oder mono- und polyfunktionelle (Meth)acrylat(e),
(b) 0,2 bis 4,0 Gew.-% und besonders bevorzugt 0,2 bis 3,0 Gew.-% mindestens einer organischen Halogenverbindung,
(c) 0,001 bis 1,0 Gew.-% und besonders bevorzugt 0,001 bis 0,1 Gew.-% mindestens einer Übergangsmetallverbindung,
(d) 0,01 bis 3,0 Gew.-% und besonders bevorzugt 0,1 bis 2,0 Gew.-% Reduktionsmittel und
(e) 22 bis 82 Gew.-% und besonders bevorzugt 35 bis 80 Gew.-% Füllstoff(e).
(f) 0,01 bis 3,0 Gew.-%, bevorzugt 0,05 bis 2,0 Gew.-% und besonders bevorzugt 0,1 bis 1,0 Gew.-% Photoinitiator, und/oder
(g) 0,001 bis 5,0 Gew.-%, bevorzugt 0,001 bis 3,0 Gew.-% und besonders bevorzugt 0,01 bis 1,0 Gew.-% Additiv(e) enthalten.

Wenn als Bestandteil (c) ein nichtkomplexes Übergangsmetallsalz eingesetzt wird, enthalten die Zusammensetzungen vorzugsweise zusätzlich 0,001 bis 1,0 Gew.-% und besonders bevorzugt 0,01 bis 1,0 Gew.-% mindestens einer komplexierenden organischen Verbindung.

Wenn nicht anders angegeben beziehen sich alle Mengenangaben auf die Gesamtmasse der Materialien. Die einzelnen Mengenbereiche können separat gewählt werden.

Besonders bevorzugt sind solche Dentalwerkstoffe, die aus den genannten Komponenten bestehen. Weiterhin sind solche Werkstoffe bevorzugt, bei denen die einzelnen Komponenten jeweils aus den oben genannten bevorzugten und besonders bevorzugten Stoffen ausgewählt sind.

Die erfindungsgemäßen Werkstoffe zeichnen sich dadurch aus, dass sie redox-chemisch härtbar sind, dabei aber keine Peroxide und vorzugsweise auch keine Weichmacher bzw. Phlegmatisierungsmittel enthalten und daher die mit diesen Stoffen verbundenen Nachteile nicht aufweisen. Außerdem haben sie eine hohe Lagerstabilität bei Raumtemperatur.

Die erfindungsgemäßen Zusammensetzungen können redox-chemisch oder, wenn ein Photoinitiator vorhanden ist, redox-chemisch und durch Licht (photo-chemisch) gehärtet werden. Vorzugsweise liegen die Werkstoffe in Form von mindestens 2, bevorzugt 2, getrennten Komponenten vor, die zur Anwendung miteinander gemischt werden. Diese Komponenten haben vornehmlich die Form von Pasten. Dabei sind die einzelnen Bestandteile des Initiatorsystems so auf die verschiedenen Komponenten verteilt, dass erst beim Mischen der Komponenten das aktive Initiatorsystem erhalten und die Polymerisation ausgelöst wird. Vorzugsweise ist die Übergangsmetallverbindung (c) in einer ersten Komponente bzw. Paste enthalten und die Halogenverbindung (c) und das Reduktionsmittel (d) in einer zweiten Komponente bzw. Paste. D.h. die erste Paste enthält vorzugsweise die Bestandteile (a), (c), (e) und ggf. einen oder mehrere der Bestandteile (f)-(g) und die zweite Komponenten die Bestandteile (a), (b), (d), (e) und ggf. einen oder mehrere der Bestandteile (f)-(g). Bei der getrennten Verwendung von Übergangsmetallsalz und Ligand können beide Bestandteile in der ersten Komponente bzw. Paste enthalten sein, oder gemäß einer anderen Ausführungsform enthält die erste Komponente das Übergangsmetallsalz und die zweite Komponente zusätzlich den Liganden. Zum Gebrauch werden die Pasten gründlich miteinander vermischt. Die Zusammensetzungen der einzelnen Komponenten bzw. Pasten sind dabei so bemessen, dass nach dem Mischen Werkstoffe mit der oben angegebenen Zusammensetzung erhalten werden. Erfindungsgemäß sind besonders solche Werkstoffe bevorzugte, die unter oralen Bedingungen (d.h. bei ca. 37 °C) gehärtet werden können.

Die erfindungsgemäßen Dentalwerkstoffe eignen sich besonders als dentale Zemente und Füllungsmaterialien. Sie haben eine sehr gute Lagerstabilität und härten dennoch schnell aus, vor allem im Vergleich zu den bekannten dibenzoylperoxidhaltigen Dentalmaterialien, und enthalten keine Peroxide und vorzugsweise auch keine Phlegmatisierungsmittel, d.h. insbesondere keine Phthalate.

Die Dentalwerkstoffe eignen sich primär zur intraoralen Anwendung durch den Zahnarzt zur Restauration geschädigter Zähne (klinische Materialien), z.B. als dentale Zemente, dentale Füllungsmaterialien und dentale Verblendmaterialien. Sie können aber auch extraoral eingesetzt werden, beispielsweise bei der Herstellung oder Reparatur von Dentalrestaurationen, wie Inlays, Onlays, Kronen und Brücken (technische Materialien).

Die Erfindung wird im Folgenden anhand von Beispielen näher erläutert.

### Ausführungsbeispiele

### Beispiel 1(Vergleichsbeispiel):

### Härtungsversuche mit füllstofffreien Härtungssystemen

Zur Untersuchung der Reaktionsfähigkeit der Redoxsysteme wurden verschiedene Härtungsversuche mit einem Redoxsystem aus BrBuE (α-Bromisobuttersäureester; organisches Halogenid), CuEH (Kupfer(II)-ethylhexanoat; Übergangsmetallverbindung), PMDETA (N,N,N',N",N"-Pentamethyldiethylen-triamin; Ligand) und SnEH (Zinn(II)-ethylhexanoat) oder AS6P (Ascorbinsäure-6-palmitat; Reduktionsmittel) untersucht. Dazu wurden 10-g-Ansätze in Schnappdeckelgläschen nach Entlüften (Exsikkator im Membranpumpenvakuum) bei Raumtemperatur durchgeführt. Als Monomere wurden 1,4-Butandioldimethacrylat (BDDMA) oder Triethylenglycoldimethacrylat (TEGDMA) eingesetzt (Tabelle 1, Angaben in Massen-%). CuEH wurde dabei jeweils erst als letzte Reaktionskomponente zugegeben. Dabei fand in den angegebenen Reaktionszeiten eine stark exotherme Polymerisation statt.

**Tabelle 1: Härtungsversuche**

| **Monomer** | **BDDMA** | **TEGDMA** | **TEGDMA** |
|---|---|---|---|
| Monomergehalt (%) | 97,63 | 97,13 | 97,60 |
| CuEH (%) | 0,04 | 0,004 | 0,0004 |
| BrBuE (%) | 1,35 | 1,46 | 1,40 |
| PMDETA (%) | 0,52 | 0,47 | 0,49 |
| SnEH | 0,47 | - | - |
| AS6P (%) | - | 0,47 | 0,47 |
| Zeit bis Reaktionsbeginn (Min) | 15 | 7 | 10 |

### Beispiel 2:

### Komposit mit einem Redoxinitiatorsystem

Mit einem Mischer (SpeedMixer^{®} DAC 150.1 FVZ; Hauschild, Deutschland), 3 Min bei 2500 Umdrehungen/Min) wurden 2 Kompositpasten mit der in Tabelle 2 angegebenen Zusammensetzung hergestellt.

**Tabelle 2: Zusammensetzung der Pasten**

| **Bestandteil** | **Paste A [Gew.-%]** | **Paste B [Gew.-%]** |
|---|---|---|
| TEGDMA | 17,58 | 17,19 |
| Bis-GMA | 17,58 | 17,19 |
| CuEH | 0,30 | - |
| BrBuE | - | 0,48 |
| PMDETA | - | 0,17 |
| AS6P | - | 0,17 |
| SiO₂¹⁾ | 44,50 | 44,80 |
| YbF₃²⁾ | 20,04 | 20,00 |
| Summe | 100,0 | 100,00 |
| ¹⁾ silanisierte pyrogene Kieselsäure, Primärpartikelgröße 40 nm (OX-50, Degussa) | | |
| ²⁾ Ytterbiumtrifluorid, mittlere Partikelgröße 0,8 µm Nach dem Mischen der Pasten im Verhältnis 1:1 wurden von den Materialien Prüfkörper gemäß ISO 4049 präpariert, die 4 h bei 80°C ausgehärtet wurden. Nach der ISO-Norm ISO-4049 (Dentistry - Polymer-based filling, restorative and luting materials) erfolgte die Bestimmung der Biegefestigkeit zu 93 MPa und des Biege-E-Moduls zu 5.700 MPa. | | |

### Beispiel 3:

### Herstellung eines Komposits mit dualhärtendem Initiatorsystem

Mit einem Mischer (Speedmixer DAC 150.1 FVZ; Fa. Hauschild, Deutschland) wurden 2 Kompositpasten mit der in Tabelle 3 angegebenen Zusammensetzung hergestellt.

**Tabelle 3: Zusammensetzung des dualhärtenden Komposits**

| **Bestandteil** | **Paste A [Gew.-%]** | **Paste B [Gew.-%]** |
|---|---|---|
| TEGDMA | 17,33 | 17,33 |
| Bis-GMA | 17,32 | 17,32 |
| CuEH | 346 ppm | - |
| BrBuE | 0,46 | 0,48 |
| PMDETA | - | 0,29 |
| AS6P | - | 0,69 |
| SiO₂¹⁾ | 44,50 | 44,58 |
| YbF₃²⁾ | 19,79 | 17,79 |
| Campherchinon | 0,21 | - |
| EMBO³⁾ | 0,36 | - |
| Summe | 100,0 | 100,00 |
| ¹⁾ silanisierte pyrogene Kieselsäure, Primärpartikelgröße 40 nm (OX-50, Degussa) | | |
| ²⁾ Ytterbiumtrifluorid, mittlere Partikelgröße 0,8 µm | | |
| ³⁾ 4-(Dimethylamino)-benzoesäureethylester | | |

Nach dem Mischen der Pasten im Verhältnis 1:1 wurden von den Materialien Prüfkörper präpariert, die zunächst 24 h bei 37 °C und dann 2 x 3 Min. im Lichtofen (Spectramat; Ivoclar Vivadent AG) ausgehärtet wurden. Die Aushärtung bei 37°C setzte nach 270 s (Verarbeitungsbreite) ein, die Aushärtungszeit betrug 320 s. Die gemessenen mechanischen Eigenschaften sind in Tabelle 4 angegeben. Die Dimension der Prüfkörper, die Bestimmung der Aushärtungszeit, der Verarbeitungszeit, der Biegefestigkeit und des Biege-E-Moduls erfolgte nach der ISO-Norm ISO-4049 (Dentistry - Polymer-based filling, restorative and luting materials).

**Tabelle 4: Mechanische Eigenschaften des dualhärtenden Komposits**

| | Biegefestigkeit (MPa) | Biege-E-Modul (GPa) |
|---|---|---|
| 37°C¹⁾ | 53,1 | 2,31 |
| 2 x 3 Min. Lichtofen | 103,3 | 6,72 |
| ¹⁾ Die Messung erfolgte gemäß ISO 4049 nach 24stündiger Lagerung bei 37°C | | |

Die Ergebnisse zeigen, dass durch Kombination eines ATRP Initiatorsystems mit einem Photoinitiatorsystem eine deutliche Verbesserung der mechanischen Eigenschaften möglich ist.

## Patentansprüche

1. Dentalwerkstoff, der
(a) 5 bis 80 Gew.-% mindestens eines di- oder polyfunktionellen radikalisch polymerisierbaren (Meth)acrylat-monomers,
(b) 0,1 bis 6,0 Gew.-% mindestens einer organischen Halogenverbindung,
(c) 0,0001 bis 1,0 Gew.-% mindestens einer Übergangsmetallverbindung,
(d) 0,01 bis 5,0 Gew.-% mindestens eines Reduktionsmittels und
(e) 10 bis 85 Gew.-% mindestens eines organischen und/oder anorganischen Füllstoffs enthält,
wobei der Dentalwerkstoff dann, wenn Bestandteil (c) ein nichtkomplexes Übergangsmetallsalz ist, zusätzlich 0,0001 bis 1,0 Gew.-% mindestens einer komplexierenden organischen Verbindung enthält und
wobei der Dentalwerkstoff keine Peroxide enthält.

2. Dentalwerkstoff nach Anspruch 1, der als Monomer (a) eine Mischung von mono- und polyfunktionellen Methacrylaten oder eine Mischung von mono- und difunktionellen Methacrylaten enthält.

3. Dentalwerkstoff nach einem der Ansprüche 1 bis 2, der als Halogenverbindung (b) mindestens eine Verbindung enthält, die aus halogenierten Alkylaromaten, Benzylhalogeniden, Benzylchlorid, Benzylbromid, 1-Phenylethylchlorid, Benzotrichlorid, Benzhydrylchlorid, Benzhydrylbromid; α-Halogencarbonsäureestern, C₁-C₆-Alkylestern einer α-Halogen-C₁-C₆-Carbonsäure, α-Chlorpropionsäure, α-Brompropionsäure, α-Chlor-iso-butansäure, α-Brom-iso-butansäure, α-Bromphenylessigsäure, Estern der α-Brom-iso-butansäure, α-Brom-isobutansäuremethylester, α-Brom-isobutansäureethylester, α-Brom-iso-butyrylbromid, 2-(2-Brom-isobutyryl-oxy)ethylmethacrylate, tert-Butyl-α-brom-iso-butyrat, 3-Butynyl-2-brom-iso-butyrat, Dipentaerythritolhexakis(2-brom-iso-butyrat), 1,1,1-Tris(2-brom-iso-butyryloxymethyl)ethan, Ethyl α-bromophenylacetat; α-Halogenketonen, 1,1,1-Trichlorpropan-2-on, Dichlormethylphenylketon; α-Halogennitrilen, 2-Brompropionitril, Trichloracetonitril; Sulfonylhalogeniden, Methylsulfonylchlorid, Trichlormethylsulfonylchlorid, p-Toluolsulfonylchlorid und 4-Methoxyphenylsulfonylchlorid ausgewählt ist.

4. Dentalwerkstoff nach einem der Ansprüche 1 bis 3, der als Komponente (c) mindestens eine Übergangsmetallkomplexverbindung enthält, die aus den Komplexen der Metalle Kupfer, Eisen, Ruthenium, Nickel und Palladium; Kupferkomplexen mit den Liganden Phenanthrolin, 1,10-Phenanthrolin (Phen), Terpyridin, Bipyridin, 2,2'-Bipyridin (Bipy), 4,4'-Dimethyl-2,2'-bipyridin, 6,6'-Dimethyl-2,2'-bipyridin, Pyridinimin, aliphatischen Aminen, 1,1,4,7,10,10-Hexamethyltriethylentetramin (HMTETA), N,N,N',N",N"-Pentamethyldi-ethylentriamin (PMDETA) und Tris[2-(dimethylamino)ethyl]-amin (Me₆TREN); Eisenkomplexen mit den Liganden Triphenylphosphin, 4,4'-Di(5-nonyl)-2,2'-bipyridin (dNbpy) oder 1,3-Diisopropyl-4,5-dimethylimidazol-2-yliden (PriIm), FeCl₂(PPh₃)₂, FeBr₂-dNbpy (dNbpy: 4,4'-Di(5-nonyl)-2,2'-bipyridin), FeCl₂(PriIm)₂ und FeBr₂(PriIm)₂ (PriIm: 1,3-Diisopropyl-4,5-dimethylimidazol-2-yliden); den Rutheniumkomplexen RuCl₂(PPh₃)₄ und RuH₂(PPh₃)₄; oder den Nickelkomplexen Ni[o,o'(CH₂NMe₂) C₆H₃]B und NiBr₂(PPh₃)₂ ausgewählt ist.

5. Dentalwerkstoff nach einem der Ansprüche 1 bis 4, der als Komponente (c) mindestens ein Übergangsmetallsalz und mindestens eine komplexierende organische Verbindung enthält, wobei
das Übergangsmetallsalz aus den Salzen der Metalle Kupfer, Eisen, Ruthenium, Nickel und Palladium, CuCl, CuBr, CuCl₂, CuBr₂, CuI₂, Cu(II)-carboxylaten, Cu(II)-Acetat, Cu(II)-2-Ethylhexanoat, FeCl₃, FeBr₂, FeCl₂, RuCl₂, NiBr₂, NiCl₂, Pd(OAc)₂ ausgewählt ist und
die komplexierende organische Verbindung aus P- und N-haltigen Liganden, Phosphinen, Triphenylphosphin; Ethylendiaminotetraessigsäure (EDTA); Phenanthrolinen, 1,10-Phenanthrolin (Phen); Bipyridinen, 2,2'-Bipyridin (Bipy), 4,4'-Dimethyl-2,2'-bipyridin, 6,6'-Dimethyl-2,2'-bipyridin und 4,4'-Di(5-nonyl)-2,2'-bipyridin (dNbpy); Terpyridinen, 2':6',2"-Terpyridin (tpy), 4,4',4"-Tris(5-nonyl)-2,2':6',2"-terpyridin; Pyridiniminen, N-Octyl-2-pyridyl-methanimin; alkylierte Imidazole, 1,3-Diisopropyl-4,5-dimethylimidazol-2-yliden (PriIm); alkylaminosubstituierten Benzolen, o,o'(CH₂NMe₂)₂C₆H₃; aliphatischen Aminen, 1,1,4,7,10,10-Hexamethyltriethylentetramin (HMTETA), N,N,N',N",N"-Pentamethyldiethylentriamin (PMDETA), Tris[2-(dimethylamino)ethyl]amin (Me₆TREN), *N,N,N',N'-*Tetramethylethylendiamin (TMEDA), 1,4,8,11-Tetraaza-1,4,8,11-tetramethylcyclotetradecan (Me4CYCLAM), Diethylentriamin (DETA), Triethylentetramin (TETA) und 1,4,8,11-Tetraazacyclotetradecan (CYCLAM)); pyridinhaltigen Liganden, N,N,N',N'-Tetrakis(2-pyridylmethyl)ethylendiamin (TPEN), N,N-Bis(2-pyridylmethyl)amin (BPMA) und N,N-Bis(2-pyridylmethyl)octylamin (BPMOA), alkylsubstituierten 2-(1H-1,2,3-Triazol-4-yl)pyridinen, 2,6-Bis(1H-1,2,3-triazol-4-yl)-pyridin und alkylsubstituierten 2,6-Bis(1H-1,2,3-triazol-4-yl)pyridinen ausgewählt ist.

6. Dentalwerkstoff nach einem der Ansprüche 1 bis 5, der als Reduktionsmittel (d) mindestens eine Verbindung enthält, die aus Ascorbinsäure und ihren Derivaten, Zinnverbindungen, Sn(II)-octoat, Sn(II)-2-ethylhexanoat, reduzierenden Zuckern, Glucose, Fructose, Antioxidantien, β-Karotinoiden, Vitamin A, α-Tocopherol (Vitamin E), phenolischen Reduktionsmitteln, Butylhydroxytoluol (BHT), Butylhydroxyanisol (BHA), Propylgallat, Octylgallat, Pyrogallol, Sulfiten, Bisulfiten, Thiosulfaten, Hydroxylamin, Hydrazin und deren Derivaten ausgewählt ist.

7. Dentalwerkstoff nach einem der Ansprüche 1 bis 6, der zusätzlich einen Photoinitiator enthält.

8. Dentalwerkstoff nach einem der Ansprüche 1 bis 7, der keine Weichmacher/Phlegmatisierungsmittel enthält.

9. Dentalwerkstoff nach einem der Ansprüche 1 bis 8, der
(a) 10 bis 70 Gew.-% und bevorzugt 15 bis 60 Gew.-% polyfunktionelle oder mono- und polyfunktionelle (Meth)acrylat(e),
(b) 0,2 bis 4,0 Gew.-% und bevorzugt 0,2 bis 3,0 Gew.-% mindestens einer organischen Halogenverbindung,
(c) 0,001 bis 1,0 Gew.-% und bevorzugt 0,001 bis 0,1 Gew.-% mindestens einer Übergangsmetallverbindung,
(d) 0,01 bis 3,0 Gew.-% und bevorzugt 0,1 bis 2,0 Gew.-% Reduktionsmittel und
(e) 22 bis 82 Gew.-% und bevorzugt 35 bis 80 Gew.-% Füllstoff(e) enthält,
wobei der Dentalwerkstoff dann, wenn Bestandteil (c) ein nichtkomplexes Übergangsmetallsalz ist, 0,001 bis 1,0 Gew.-% und bevorzugt 0,01 bis 1,0 Gew.-% mindestens einer komplexierenden organischen Verbindung enthält.

10. Dentalwerkstoff nach einem der Ansprüche 1 bis 9, der zusätzlich
(f) 0,01 bis 3,0 Gew.-%, bevorzugt 0,05 bis 2,0 Gew.-% und besonders bevorzugt 0,1 bis 1,0 Gew.-% Photoinitiator, und/oder
(g) 0,001 bis 5,0 Gew.-%, bevorzugt 0,001 bis 3,0 Gew.-% und besonders bevorzugt 0,01 bis 1,0 Gew.-% Additiv(e) enthält.

11. Dentalwerkstoff nach einem der Ansprüche 1 bis 10, der in Form von mindestens zwei getrennten Komponenten vorliegt, die vor der Anwendung miteinander gemischt werden.

12. Dentalwerkstoff nach Anspruch 11, der eine erste Komponente und eine zweite Komponente umfasst, wobei die erste Komponente die Bestandteile (a), (c), (d) und ggf. einen oder mehrere der Bestandteile (f)-(g) und die zweite Komponenten die Bestandteile (a), (b), (d), (e) und ggf. einen oder mehrere der Bestandteile (f)-(g) enthält.

13. Dentalwerkstoff nach einem der Ansprüche 1 bis 12 zur intraoralen Verwendung als Zement oder Füllungsmaterial bei der Restauration geschädigter Zähne.

14. Verwendung eines Dentalwerkstoffs gemäß einem der Ansprüche 1 bis 12 zur extraoralen Herstellung oder Reparatur von Dentalrestaurationen, Inlays, Onlays, Kronen oder Brücken.

## Claims

1. Dental material which contains
(a) 5 to 80 wt.% of at least one di- or polyfunctional radically polymerisable (meth)acrylate monomer,
(b) 0.1 to 6.0 wt.% of at least one organic halogen compound,
(c) 0.0001 to 1.0 wt.% of at least one transition metal compound,
(d) 0.01 to 5.0 wt.% of at least one reducing agent, and
(e) 10 to 85 wt.% of at least one organic and/or inorganic filler,
wherein, if constituent (c) is a non-complex transition metal salt, the dental material additionally contains 0.0001 to 1.0 wt.% of at least one complexing organic compound, and
wherein the dental material does not contain peroxides.

2. Dental material according to claim, which contains as monomer (a) a mixture of mono- and polyfunctional methacrylates or a mixture of mono- and difunctional methacrylates.

3. Dental material according to any one of the claims 1 to 2 which contains as halogen compound (b) at least one compound which is selected from halogenated alkylaromatics, benzyl halides, benzyl chloride, benzyl bromide, 1-phenylethyl chloride, benzotrichloride, benzhydryl chloride, benzhydryl bromide; α-halogen carboxylic acid esters, C₁-C₆-alkyl esters of an α-halogen-C₁-C₆-carboxylic acid, α-chloropropionic acid, α-bromopropionic acid, α-chloro-iso-butanoic acid, α-bromo-iso-butanoic acid, α-bromophenylacetic acid, esters of α-bromo-iso-butanoic acid, α-bromo-isobutanoic acid methyl ester, α-bromo-isobutanoic acid ethyl ester, α-bromo-isobutyryl bromide, 2-(2-bromoisobutyryloxy)ethyl methacrylate, tert-butyl α-bromoisobutyrate, 3-butynyl-2-bromo-iso-butyrate, dipentaerythritol hexakis(2-bromoisobutyrate), 1,1,1-tris(2-bromoisobutyryloxymethyl)-ethane, ethyl α-bromophenylacetate; α-halogenketones, 1,1,1-trichloropropan-2-one, dichloromethyl phenyl ketone; α-halogen nitriles, 2-bromopropionitrile, trichloroacetonitrile; sulphonyl halides, methylsulphonyl chloride, trichloromethylsulphonyl chloride, p-toluenesulphonyl chloride and 4-methoxyphenylsulphonyl chloride.

4. Dental material according to any one of the claims 1 to 3 which contains as component (c) at least one transition metal complex compound which is selected from the complexes of the metals copper, iron, ruthenium, nickel and palladium; copper complexes with the ligands phenanthroline, 1,10-phenanthroline (Phen), terpyridine, bipyridine, 2,2'-bipyridine (Bipy), 4,4'-dimethyl-2,2'-bipyridine, 6,6'-dimethyl-2,2'-bipyridine, pyridinimine, aliphatic amines, 1,1,4,7,10,10-hexamethyltriethylenetetramine (HMTETA), N,N,N',N'',N''-pentamethyldiethylene-triamine (PMDETA) and tris[2-(dimethylamino)ethyl]amine (Me₆TREN); iron complexes with the ligands triphenylphosphine, 4,4'-di(5-nonyl)-2,2'-bipyridine (dNbpy) or 1,3-diisopropyl-4,5-dimethylimidazol-2-ylidene (PriIm), FeCl₂(PPh₃)₂, FeBR₂-dNbpy (dNbpy: 4,4'-di(5-nonyl)-2,2'-bipyridine), FeCl₂(PriIm)₂ and FeBr₂(PriIm)₂ (PriIm: 1,3-diisopropyl-4,5-dimethylimidazol-2-ylidene); the ruthenium complexes RuCl₂(PPh₃)₄ and RuH₂(PPh₃)₄; or the nickel complexes Ni [o, o' (CH₂NMe₂) C₆H₃] Br and NiBr₂ (PPh₃)₂.

5. Dental material according to any one of the claims 1 to 4 which contains as component (c) at least one transition metal salt and at least one complexing organic compound,
wherein the transition metal salt is selected from the salts of the metals copper, iron, ruthenium, nickel, and palladium, CuCl, CuBr, CuCl₂, CuBr₂, CuI₂, Cu(II) carboxylates, Cu(II) acetate, Cu(II)-2-ethylhexanoate, FeCl₃, FeBr₂, FeCl₂, RuCl₂, NiBr₂, NiCl₂, Pd(OAc)₂ and
the complexing organic compound is selected from P- and N-containing ligands, phosphines, triphenylphosphine; ethylenediaminetetraacetic acid (EDTA); phenanthrolines, 1,10-phenanthroline (Phen); bipyridines, 2,2'-bipyridine (Bipy), 4,4'-dimethyl-2,2'-bipyridine, 6,6'-dimethyl-2,2'-bipyridine and 4,4'-di(5-nonyl)-2,2'-bipyridine (dNbpy); terpyridines, 2':6',2"-terpyridine (tpy), 4,4',4"-tris(5-nonyl)-2,2':6',2"-terpyridine; pyridinimines, N-octyl-2-pyridylmethanimine; alkylated imidazoles, 1,3-diisopropyl-4,5-dimethylimidazol-2-ylidene (PriIm); alkylamino-substituted benzenes, o,o'(CH₂NMe₂) ₂C₆H₃; aliphatic amines, 1,1,4,7,10,10-hexamethyltriethylenetetramine (HMTETA), N,N,N',N",N"-pentamethyldiethylenetriamine (PMDETA), tris-[2-(dimethylamino)ethyl]amine (Me₆TREN), N,N,N',N'-tetramethylethylenediamine (TMEDA), 1,4,8,11-tetraaza-1,4,8,11-tetramethylcyclotetradecane (Me4CYCLAM), diethylenetriamine (DETA), triethylenetetramine (TETA) and 1,4,8,11-tetraazacyclotetradecane (CYCLAM)); pyridine-containing ligands, N,N,N',N'-tetrakis(2-pyridylmethyl)ethylenediamine (TPEN), N,N-bis(2-pyridylmethyl)amine (BPMA) and N,N-bis(2-pyridylmethyl) octylamine(BPMOA), alkyl-substituted 2-(1H-1,2,3-triazol-4-yl)pyridines, 2,6-bis-(1H-1,2,3-triazol-4-yl)-pyridine and alkyl-substituted 2,6-bis(1H-1,2,3-triazol-4-yl)pyridines.

6. Dental material according to any one of the claims 1 to 5 which contains as reducing agent (d) at least one compound which is selected from ascorbic acid and derivatives thereof, tin compounds, Sn(II) octoate, Sn(II)-2-ethylhexanoate, reducing sugars, glucose, fructose, antioxidants, β-carotenoids, vitamin A, α-tocopherol (vitamin E), phenolic reducing agents, butylhydroxytoluene (BHT), butylhydroxyanisole (BHA), propyl gallate, octyl gallate, pyrogallol, sulphites, bisulphites, thiosulphates, hydroxylamine, hydrazine and derivatives thereof.

7. Dental material according to any one of the claims 1 to 6, which additionally contains a photoinitiator.

8. Dental material according to one any of the claims 1 to 7 which contains no peroxides and/or plasticisers/phlegmatising agents.

9. Dental material according to any one of the claims 1 to 8, which contains
(a) 10 to 70 wt.%, and preferably 15 to 60 wt.% polyfunctional or mono- and polyfunctional (meth)acrylate(s),
(b) 0.2 to 4.0 wt.%, and preferably 0.2 to 3.0 wt.% of at least one organic halogen compound,
(c) 0.001 to 1.0 wt.%, and preferably 0.001 to 0.1 wt.% of at least one transition metal compound,
(d) 0.01 to 3.0 wt.%, and preferably 0.1 to 2.0 wt.% reducing agent, and
(e) 22 to 82 wt.%, and preferably 35 to 80 wt.% filler(s),
wherein, if constituent (c) is a non-complex transition metal salt, the dental material contains 0.001 to 1.0 wt.%, and preferably 0.01 to 1.0 wt.% of at least one complexing organic compound.

10. Dental material according to any one of the claims 1 to 9, which additionally contains
(f) 0.01 to 3.0 wt.%, preferably 0.05 to 2.0 wt.%, and particularly preferably 0.1 to 1.0 wt.% photoinitiator, and/or
(g) 0.001 to 5.0 wt.%, preferably 0.001 to 3.0 wt.% and particularly preferably 0.01 to 1.0 wt.% additive(s).

11. Dental material according to any one of the claims 1 to 10 which is present in the form of at least two separate components which are mixed together before use.

12. Dental material according to claim 11 which comprises a first component and a second component, wherein the first component contains the constituents (a), (c), (d), and optionally one or more of the constituents (f)-(g) and the second component contains the constituents (a), (b), (d), (e), and optionally one or more of the constituents (f)-(g).

13. Dental material according to any one of the claims 1 to 12 for intraoral use as cement or filling material in the restoration of damaged teeth.

14. Use of a dental material according to any one of the claims 1 to 12 for extraoral manufacturing or repairing dental restorations, inlays, onlays, crowns, or bridges.

## Revendications

1. Matériau dentaire, qui contient
(a) 5 à 80 % en poids d'au moins un monomère (méth)acrylate bi- ou polyfonctionnel polymérisable par voie radicalaire,
(b) 0,1 à 6,0 % en poids d'au moins un composé halogéné organique,
(c) 0,0001 à 1,0 % en poids d'au moins un composé de métal de transition et
(d) 0,01 à 5,0 % en poids d'au moins un agent de réduction, et
(e) 10 à 85 % en poids d'au moins une charge organique et/ou inorganique,
dans lequel le matériau dentaire contient, lorsque l'élément constitutif (c) est un sel de métal de transition non complexe, en supplément 0,0001 à 1,0 % en poids d'au moins un composé organique complexant, et
dans lequel le matériau dentaire ne contient pas de peroxydes.

2. Matériau dentaire selon la revendication 1, qui contient comme monomère (a) un mélange de méthacrylates mono- et polyfonctionnels ou un mélange de méthacrylates mono- et bifonctionnels.

3. Matériau dentaire selon l'une quelconque des revendications 1 à 2, qui contient comme composé halogéné (b) au moins un composé, qui est sélectionné parmi les composés alkyles aromatiques halogénés, halogénures de benzyle, chlorure de benzyle, bromure de benzyle, 1-phényléthylchlorure, benzotrichlorure, chlorure de benzhydryle, bromure de benzhydryle ; esters d'acide α-halogénocarboxylique, esters d'alkyle en C₁-C₆ d'un acide α-halogénocarboxylique en C₁-C₆, acide α-chloropropionique, acide α-bromopropionique, acide α-chloro-iso-butanoïque, acide α-bromo-iso-butanoïque, acide α-bromophénylacétique, esters de l'acide α-bromo-iso-butanoïque, méthylester d'acide α-bromo-isobutanoïque, éthylester d'acide α-bromo-iso-butanoïque, bromure α-bromo-iso-butyryle, 2-(2-bromo-isobutyryloxy)éthylméthacrylate, tert-butyl-α-bromo-iso-butyrate, 3-butynyl-2-bromo-iso-butyrate, dipentaérythritolhexakis(2-bromo-iso-butyrate), 1,1,1-tris(2-bromo-iso-butyryloxyméthyl)éthane, éthyle α-bromophénylacétate ; α-halogénocétones, 1,1,1-trichloropropan-2-one, dichlorométhylphenylcétone ; α-nitriles halogénés, 2-bromopropionitrile, trichloroacétonitrile ; halogénures de sulfonyle, chlorure de méthylsulfonyle, chlorure de trichlorométhylsulfonyle, chlorure de p-toluènesulfonyle et chlorure de 4-méthoxyphénylsulfonyle.

4. Matériau dentaire selon l'une quelconque des revendications 1 à 3, qui contient comme composant (c) au moins un composé complexe de métal de transition, qui est sélectionné parmi les complexes des métaux cuivre, fer, ruthénium, nickel et palladium ; complexes de cuivre avec les ligands phénanthroline, 1,10-phénanthroline (phen), terpyridine, bipyridine, 2,2'-bipyridine (bipy), 4,4'-diméthyl-2,2'-bipyridine, 6,6'-diméthyl-2,2'-bipyridine, pyridinimine, amines aliphatiques, 1,1,4,7,10,10-hexaméthyltriéthylènetétramine (HMTETA), N,N,N',N",N"-pentaméthyldiéthylènetriamine (PMDETA) et tris[2-(diméthylamino)éthyl]amine (Me₆TREN) ; complexes de fer avec les ligands triphénylphosphine, 4,4'-di(5-nonyl)-2,2'-bipyridine (dNbpy) ou 1,3-diisopropyl-4,5-diméthylimidazol-2-ylidène (PriIm), FeCl₂(PPh₃)₂, FeBR₂-dNbpy (dNbpy : 4,4'-di(5-nonyl)-2,2'-bipyridine), FeCl ₂(PriIm)₂ et FeBr₂(PriIm)₂ (PriIm : 1,3-diisopropyl-4,5-diméthylimidazol-2-ylidène) ; les complexes de ruthénium RuCl₂(PPh₃)₄ et RuH₂(PPh₃)₄ ; ou les complexes de nickel Ni [o,o'(CH₂NMe₂) C₆H₃]B et NiBr₂ (PPh₃)₂.

5. Matériau dentaire selon l'une quelconque des revendications 1 à 4, qui contient comme composant (c) au moins un sel de métal de transition et au moins un composé organique complexant, dans lequel
le sel de métal de transition est sélectionné parmi les sels des métaux cuivre, fer, ruthénium, nickel et palladium, CuCl, CuBr, CuCl₂, CuBr₂, CuI₂, Cu(II)-carboxylates, Cu(II)-acétate, Cu(II)-2-éthylhexanoate, FeCl₃, FeBr₂, FeCl₂, RuCl₂, NiBr₂, NiCl₂, Pd(OAc)₂ et
le composé organique complexant est sélectionné parmi les ligands contenant P et N, phosphines, triphénylphosphine ; acide éthylènediaminetétraacétique (EDTA) ; phénanthrolines, 1,10-phénanthroline (phen) ; bipyridines, 2,2'-bipyridine (bipy), 4,4'-diméthyl-2,2'-bipyridine, 6,6'-diméthyl-2,2'-bipyridine et 4,4'-di(5-nonyl)-2,2'-bipyridine (dNbpy) ; terpyridines, 2':6',2"-terpyridine (tpy), 4,4',4"-tris(5-nonyl)-2,2':6',2"-terpyridine ; pyridinimines, N-octyl-2-pyridylméthanimine ; imidazoles alkylés, 1,3-diisopropyl-4,5-diméthylimidazol-2-ylidène (PriIm) ; benzènes substitués par alkylamino, o, o'(CH₂NMe₂) ₂C₆H₃ ; amines aliphatiques, 1,1,4,7,10,10-hexaméthyltriéthylènetétramine (HMTETA), N,N,N',N",N"-pentaméthyldiéthylènetriamine (PMDETA), tris-[2-(diméthylamino)éthyl]amine (Me₆TREN), N,N,N',N'-tétraméthyléthylènediamine (TMEDA), 1,4,8,11-tétraaza-1,4,8,11-tétraméthylcyclotétradécane (Me4CYCLAM), diéthylènetriamine (DETA), triéthylènetétramine (TETA) et 1,4,8,11-tétraazacyclotétradécane (CYCLAM) ; ligands contenant de la pyridine, N,N,N',N'-tétrakis(2-pyridylméthyl)éthylènediamine (TPEN), N,N-bis(2-pyridylméthyl)amine (BPMA) et N,N-bis(2-pyridylméthyl)octylamine (BPMOA), 2-(1H-1,2,3-triazol-4-yl)pyridines substitués par alkyle, 2,6-bis(1H-1,2,3-triazol-4-yl)-pyridine et 2,6-bis(1H-1,2,3-triazol-4-yl)pyridines substitués par alkyle.

6. Matériau dentaire selon l'une quelconque des revendications 1 à 5, qui contient comme agent de réduction (d) au moins un composé, qui est sélectionné parmi l'acide ascorbique et ses dérivés, les composés d'étain, Sn(II)-octoate, Sn(II)-2-éthylhexanoate, sucres réducteurs, glucose, fructose, antioxydants, β-caroténoïdes, vitamine A, α-tocophérol (vitamine E), agents de réduction phénoliques, butylhydroxytoluène (BHT), butylhydroxyanisole (BHA), propylgallate, octylgallate, pyrogallol, sulfites, bisulfites, thiosulfates, hydroxylamine, hydrazine et leurs dérivés.

7. Matériau dentaire selon l'une quelconque des revendications 1 à 6, qui contient en plus un photoinitiateur.

8. Matériau dentaire selon l'une quelconque des revendications 1 à 7, qui ne contient pas de plastifiants/flegmatisants.

9. Matériau dentaire selon l'une quelconque des revendications 1 à 8, qui contient
(a) 10 à 70 % en poids et de manière préférée 15 à 60 % en poids de (méth)acrylate(s) polyfonctionnel(s) ou mono- et polyfonctionnel(s),
(b) 0,2 à 4,0 % en poids et de manière préférée 0,2 à 3,0 % en poids d'au moins un composé halogéné organique,
(c) 0,001 à 1,0 % en poids et de manière préférée 0,001 à 0,1 % en poids d'au moins un composé de métal de transition,
(d) 0,01 à 3,0 % en poids et de manière préférée 0,1 à 2,0 % en poids d'agents de réduction et
(e) 22 à 82 % en poids et de manière préférée 35 à 80 % en poids de charge(s),
dans lequel le matériau dentaire contient alors, lorsque l'élément constitutif (c) est un sel de métal de transition non complexe, 0,001 à 1,0 % en poids et de manière préférée 0,01 à 1,0 % en poids d'au moins un composé organique complexant.

10. Matériau dentaire selon l'une quelconque des revendications 1 à 9, qui contient en plus
(f) 0,01 à 3,0 % en poids, de manière préférée 0,05 à 2,0 % en poids et de manière particulièrement préférée 0,1 à 1,0 % en poids de photoinitiateur, et/ou
(g) 0,001 à 5,0 % en poids, de manière préférée 0,001 à 3,0 % en poids et de manière particulièrement préférée 0,01 à 1,0 % en poids d'additif(s).

11. Matériau dentaire selon l'une quelconque des revendications 1 à 10, qui est présent sous forme d'au moins deux composants séparés, qui sont mélangés l'un avec l'autre avant l'utilisation.

12. Matériau dentaire selon la revendication 11, qui comprend un premier composant et un deuxième composant, dans lequel le premier composant contient les éléments constitutifs (a), (c), (d) et éventuellement un ou plusieurs des éléments constitutifs (f)-(g) et le deuxième composant contient les éléments constitutifs (a), (b), (d), (e) et éventuellement un ou plusieurs des éléments constitutifs (f)-(g).

13. Matériau dentaire selon l'une quelconque des revendications 1 à 12 pour l'utilisation intraorale comme ciment ou matériau d'obturation lors de la restauration de dents abîmées.

14. Utilisation d'un matériau dentaire selon l'une quelconque des revendications 1 à 12 pour la fabrication extraorale ou réparation de restaurations dentaires, inlays, onlays, couronnes ou bridges.
